# EUROPEAN PATENT SPECIFICATION

(11) **EP 1 605 988 B1**
(45) Date of publication and mention of the grant of the patent: **13.08.2014**
(21) Application number: 04721776.5
(22) Date of filing: 19.03.2004
(51) Int. Cl.: A61M 1/12

(54) **CANNULA**
KANÜLE
CANULE

(30) Priority: 21.03.2003 AU 2003901345
(43) Date of publication of application: 21.12.2005
(73) Proprietor: Thoratec Corporation, Pleasanton, CA 94588 (US)
(72) Inventor: WOODARD, John, Campbell, Thornleigh, NSW 2120 (AU); BEGG, John, Donald, Forestville, NSW 2087 (AU); AYRE, Peter, Joseph, Crows Nest, NSW 2065 (AU); ALDER, Anthony, Philip, Newtown, NSW 2042 (AU)
(74) Representative: ZBM Patents - Zea, Barlocci & Markvardsen
(86) International application number: PCT/AU2004/000340
(87) International publication number: WO 2004/082742

(56) References cited:
- EP-A2- 0 232 074
- WO-A2-00/12148
- US-A- 4 086 665
- US-A- 4 639 252
- US-A- 5 314 418
- US-A- 5 984 908
- US-A- 6 001 056
- US-A- 6 152 911
- US-B1- 6 186 999
- US-B1- 6 186 999

## Description

### FIELD OF THE INVENTION

The present invention relates to a cannula assembly, and more particularly to a cannula assembly suitable for the transport of blood, preferably, between a patient's heart or circulatory system and an implantable blood pump device.

### BACKGROUND OF THE INVENTION

Blood pumps are commonly used to treat congestive heart failure and other problems associated with patient's circulatory systems and typically require connection to a heart and a circulatory system via cannulae.

To be effective, these cannulae should include a number of physical properties, among which are the abilities to flex without kinking and to resist collapse from any partial vacuum, particularly wherein a partial vacuum is induced within the cannula by a blood pump.

Additionally, it is preferable to clamp the cannula so as to allow the blood channel to be closed. This is particularly useful during installation of medical devices as clamping of the cannula permits access to the blood pump to which it is connected. This clamping should preferably be accomplished without damaging the cannula or rupturing the blood channel within the cannula.

Typically, current cannulae are formed with an inner blood channel or lumen surrounded by reinforcing spring or spring-like sheaths to reinforce the lumen. The spring-like sheaths make clamping of these types of cannulae problematic.

Additionally if the spring-like sheath is internal and disposed within the cannula, the spring-like sheaths may increase the possibility of thrombi forming. This is because the spring-like sheaths function as discontinuities within the lumen of the cannula and become sites for thrombogenesis.

US Patent 4,086,665 describes a cannula wherein the body of cannula includes a flexible polyester fabric tube with a convoluted wall structure which allows the cannula to be bent without kinking. However the fabric polyester tube Is surrounded and supported by a series of rigid rings and an exterior impervious tube. The cannula described includes continuous metallic reinforcing. A disadvantage of this cannula is that the reinforcing prevents the cannula from being selectively clamped during implantation without damaging the cannula.

US Patent 6,001,056 describes a cannula wherein the body of cannula includes a flexible tubular inner wall member which forms a blood channel and is covered with a relatively rigid cage. The relatively rigid cage gives the cannula structure support to prevent collapse, when a suction force is applied to said blood channel. The relatively rigid cage also allows the cannula sufficient flexibility to bend or deform in respect to shape. However, the body of this cannula is not capable of being clamped without significant damage resulting to the cannula and this is of significant disadvantage when attempting to implant said cannula assembly.

It is an object of the present invention to address or ameliorate at least one of the above disadvantages.

### BRIEF DESCRIPTION OF INVENTION

According to an aspect of the invention, there is provided a cannula as in claim 1.

The flexible tubular shank may have an outer portion having a hardness of between 50 to 65 Shore. The outer portion of said tubular shank may form an outer sheath, and said flexible tubular shank further may comprise an inner sheath having a substantially lesser hardness than said outer sheath. The inner sheath preferably has a hardness of about 35 Shore.

The first end includes an adaptor for connection to a heart and may include a second end adapted for connection to a blood pump. The adaptor is substantially funnel-shaped and may be pliable, and may be adapted for insertion thereof into a cored hole within the heart. The adaptor may also include a textured material mounted on an outer surface thereof.

The shank may include: at least one positioning strip; at least one radiopaque region; a series of markings along its length so as to allow determination of length of the shank inserted within the heart, when implanted; a resiliently pliable strip that allows shape retention of said shank subsequent to it being deformed; and/or at least one sensor to detect and/or measure blood flow and/or blood pressure within said cannula, when in use.

The second end may include a connector with a locking nut having an outer surface adapted to releasably engage with a corresponding connector on said blood pump.

The outer portion of said flexible tubular shank is preferably made of silicone. A flexible reinforcing material may also be moulded integrally within said flexible tubular shank.

### BRIEF DESCRIPTION OF THE DRAWINGS

Embodiments of the present invention will now be described with reference to the accompanying drawings wherein:
Fig.1 is a side view of a first preferred embodiment of a cannula assembly according to the present invention;
Fig. 2 is a side view of a second preferred embodiment of a cannula assembly according to the present invention;
Fig. 3 is a side view of a third preferred embodiment of a cannula assembly according to the present invention;
Fig. 4 is a perspective view of a fourth preferred embodiment of a cannula assembly according to the present invention;
Fig. 5 is a view of said fourth preferred embodiment, shown in Fig. 4, in situ;
Fig. 6 is an enlarged view of said fourth preferred embodiment, shown in Fig. 4, in situ;
Fig. 7 is a side view of a fifth preferred embodiment of the cannula assembly according to the present Invention;
Fig. 8 is an enlarged cross-sectional view of the preferred embodiment shown in

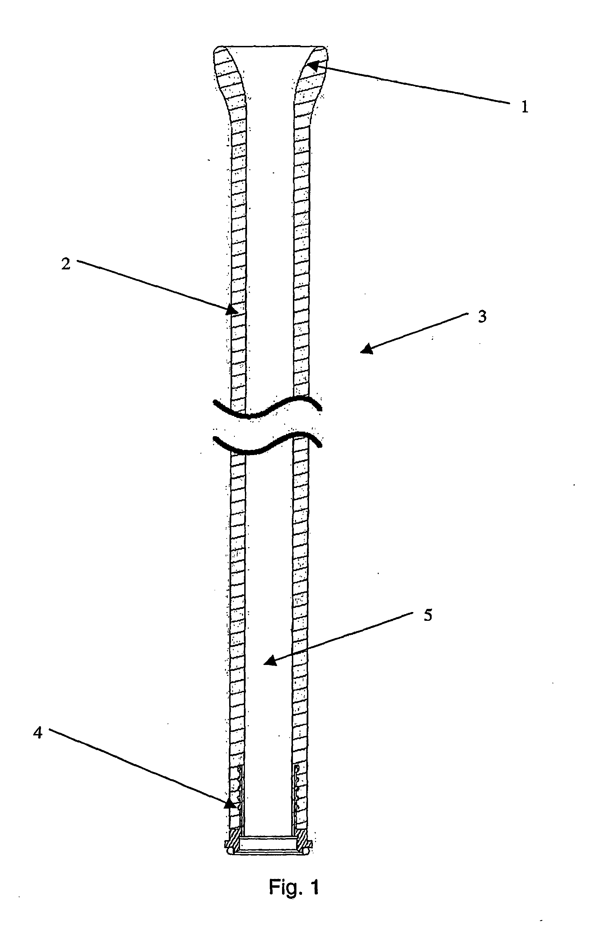

Fig. 1 wherein cannula is attached to a blood pump spigot; and

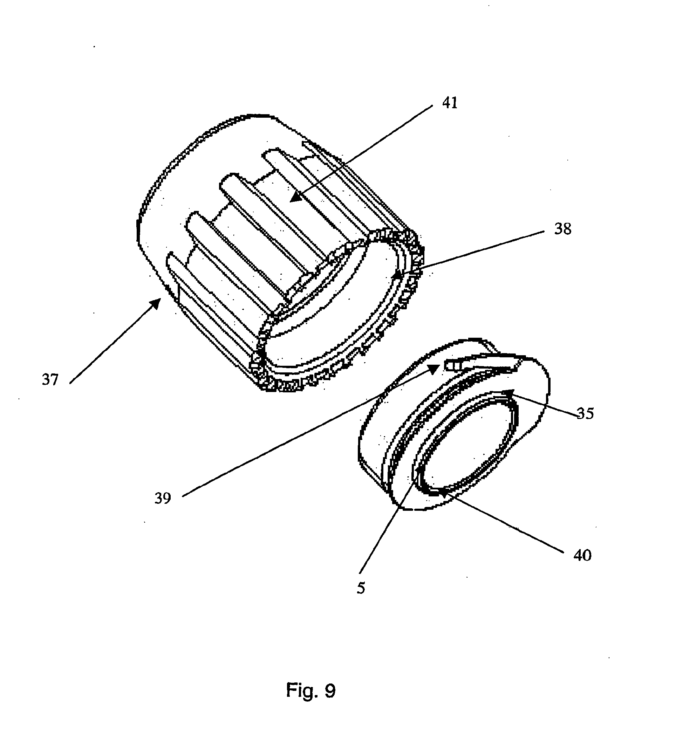

Fig. 9 is a perspective view of a portion of a further preferred embodiment.

### DETAILED DESCRIPTION OF PREFERRED EMBODIMENTS

A first preferred embodiment of the present invention is an inflow cannula for connection between the left ventricle of a heart and a blood pump. This embodiment will now be described with reference to some of the drawings.

With reference to Fig. 1, a cannula assembly 3 includes: an adaptor 1 in the form of a substantially funnel-shaped (or flared) end, a blood pump connector 4, and an elongate tubular shank comprising a thick lumen 2 that defines a blood channel 5 through the assembly 3. Whilst adaptor 1 is shown with a funnel-shaped end having a convex inner surface, it may in another not shown embodiment be other than convex.

Preferably, the adaptor 1 and the thick lumen 2 are integrally moulded from a medical grade of silicone rubber such as Nusil™. The adaptor 1 is flexible and pliable so as to allow deformation of the adaptor 1 for insertion through a cored hole in the heart of a patient. Preferably, the cored hole is made in the apex of the left ventricle of the heart.

The thick lumen 2 may preferably include bends, which may be formed during a moulding process. These bends may allow the elongate tubular shank to be custom-made to facilitate adjustment of the cannula to suit individual patient needs (please note that this feature is not shown in the accompanying figures). For clarity and ease of description the elongate tubular shanks shown in the accompanying figures are shown in a substantially straight or linear configuration.

Thick lumen 2 preferably has an outer surface, that substantially along its length has a hardness, expressed in durometers, in the range of 50-65 Shore. This allows for the elongate tubular shank of the cannula assembly to be clamped without damage, whilst still allowing a degree of flexure of the cannula without kinking.

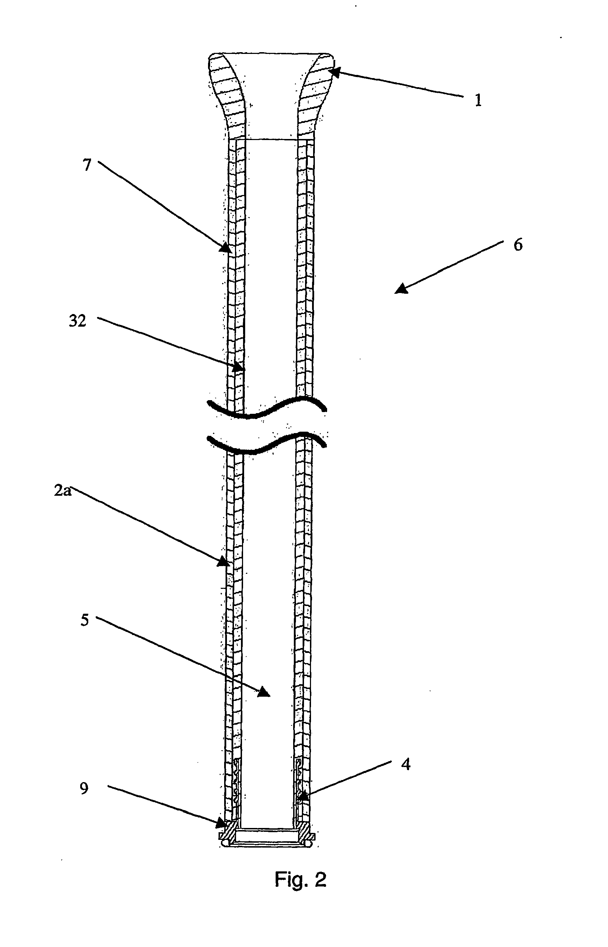

Fig. 2 shows a cannula assembly 6 including a lumen 2a which comprises an inner sheath 32 and an outer sheath 7. Preferably the inner sheath 32 is relatively stiff in comparison with adaptor 1. The hardnesses of the inner and outer sheaths 7,32 are selectable when moulded. A desired variation in stiffness between the inner sheath 32 and outer sheath 7 is achieved by selecting mouldable materials of different hardnesses. For instance, both the inner sheath 32 and outer sheath 7 may be of silicone rubber, however the material used for the inner sheath 32 having a hardness greater than the material used for the outer sheath 7.

Cannula assembly 6 shows the result of moulding a silicone outer sheath 7 to a silicone inner sheath 32, wherein said outer sheath 7 is of a lesser hardness than the inner sheath 32. The preferred hardnesses, expressed as durometers, may be approximately 65-Shore for the inner sheath 32 and approximately 35-Shore for outer sheath 7, respectively. Typically, wherein the combined hardness of the inner and outer sheathes 32, 7 to form the shank of the cannula assembly is preferably in the range of 50 - 65 Shore.

Although moulded in separate operations, the outer surface of the inner sheath 32 and the inner surface of the outer sheath 7 spontaneously bond together such that there is no movement between their adjoining surfaces or a reduced likelihood of delamination, in use. This laminated structure of the stiffer inner sheath 32 and the softer outer sheath 7, allows a greater degree of flexure of the cannula without kinking. Also, this feature may allow for clamping of the cannula at any desired point along the length of the elongate tubular shank of this embodiment, if so required during its installation. Preferably, the cannula assembly may be long lasting, resistant to chemical decomposition by a patient's body and resistant to collapse when negative pressure is applied to the blood channel.

The inner sheath 32 preferably includes a smooth and non-convoluted inner wall or surface and this inner surface, in situ, contacts the blood. The inner surface also forms a relatively smooth walled blood channel which may prevent or reduce the risk of thrombogenesis or blood clots from forming.

The adaptor 1 may also include an outer textured surface (not shown in the accompanying Figs.). This outer textured surface may permit the ingrowth of tissue when the adaptor 1 is implanted. The ingrowth of tissue may allow for better bonding between the patient and the cannula; and may also reduce risk of infection. The outer textured surface is not limited to being on the outer surface of the adaptor 1, but may also be included on the outer surface of at least a portion of the shank of the cannula.

As shown in Fig. 2, adaptor 1 has its outer surface formed to adapt to varying myocardial thicknesses while the annular form and resilience of its end is adapted to provide myocardial stenting.

Preferably, the blood pump connector 4 is attached by moulding to the lumen 2a. The moulding process is so arranged that the inner surface of the inner sheath 32 extends through the cylindrical barrel portion of connector 4 ending in close proximity to the outer face of the retaining shoulder 9. Thus the inner surface of the inner sheath 32 is a continuum from its outer end at the connector 4 to the adaptor 1, thereby minimising discontinuities which could contribute to the formation of thrombi.

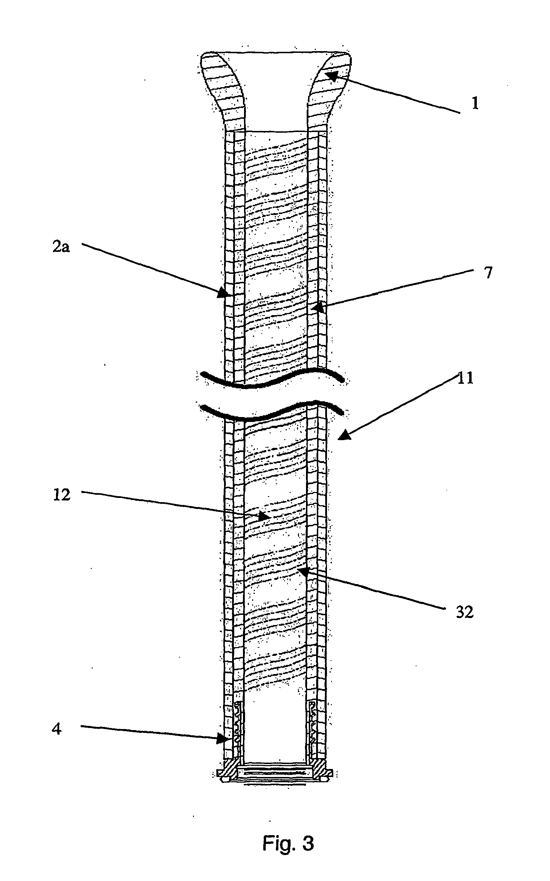

The cannula assembly may also be moulded in two stages. With reference to Fig. 3, in another preferred embodiment of the invention, the lumen 2a is impregnated with a winding of flexible reinforcing material 12 that is moulded integrally within the lumen 2a that is also capable of being clamped. The flexible reinforcing material is preferably constructed of substances such as Kevlar™. In a further preferred embodiment of the present invention, the method of manufacture employed is an injection moulding system. In this embodiment, preferably, multiple cavities defining the first stage for the formation of the inner sheath 32 and the adaptor 1 are filled under pressure with uncured silicone elastomer. When cured the product of the first moulding stage is transferred to a second preferably multiple cavity injection die for the moulding of the outer sheath 7.

Alternately, in another preferred embodiment, wherein the shank comprises a dual sheath lumen, a flexible reinforcing material may be wrapped around the outer surface of the inner sheath before the commencement of a second moulding of the outer sheath. This may have the effect of embedding the reinforcing material between the sheaths comprising the shank of this embodiment.

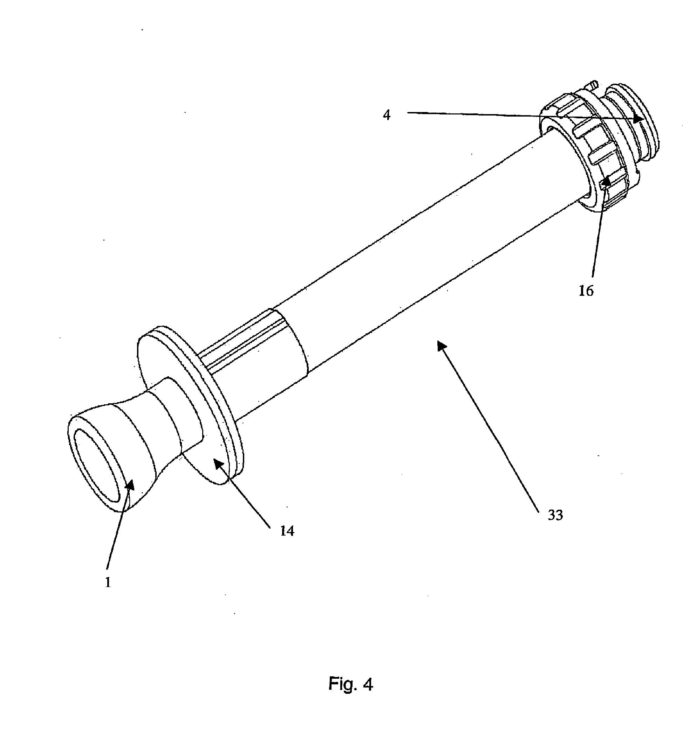

According to a further embodiment 33 shown in Fig. 4, locking nut 16 and sewing ring 14 may be slidably mounted on the cannula assembly. The cannula may be attached to the heart by first coring a suitable sized hole in the apex of the left ventricle or other suitable location and then inserting funnel-shaped end 1 into the heart a distance of approximately 20mm, or as required by surgical technique into the heart. The sewing ring 14 may then be slid up the cannula until it touches the heart where it is snared around the shank of the cannula and then sewn to a ring of pledgets placed around the base of the ventricle. The adherence of the pledgets to the myocardium may be augmented by the use of fast curing bio-glue.

Please note that the sewing ring 14 is preferably constructed of velour or plastic. If the sewing ring 14 is constructed of a relatively hard or rigid material (such as relatively rigid plastic), the sewing ring 14 may include suture h oles (not shown). Please note that these suture holes are not necessary if th e sewing ring 14 is constructed of a malleable material, such as polyester velour. Preferably, the sewing ring may be able to form an apical shape to suit the corresponding surface of the heart which the sewing ring engages.

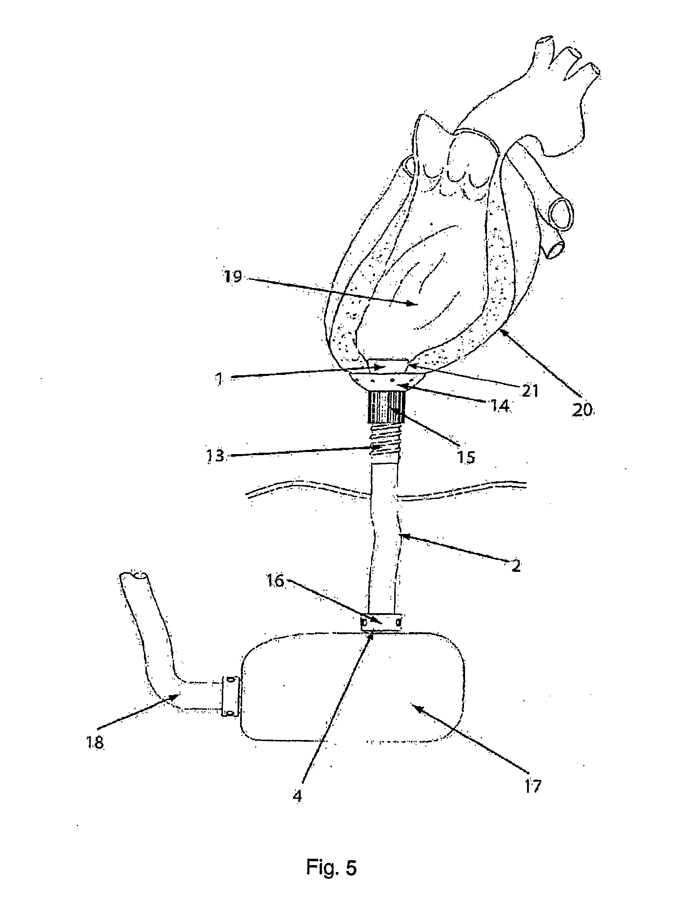

It is also preferred that the previously described embodiments may be attached to a heart and a blood pump. Fig. 5 shows the embodiment of Fig. 4, wh erein adaptor 1 is fixably inserted with the heart 20 in position to receive blood from the apex of the left ventricle 19. The distal end of the cannula is fixably connected to a blood pump 17 and is secured by locking nut 16. An outflow cannula 18 is connected to the blood pump 17. The blood pump is preferably a non-pulsatile or continuous flow heart assist device. Such heart assist devices generally demand a relatively constant flow and/or pressure and therefore cannula connected to them may be designed to not collapse under relatively continuous blood flows and/or pressures.

The adaptor 1, when implanted within a patient's ventricle, has the effect of stenting the interior walls of the ventricle away from the inlet of cannula assembly 33. Thereby, the adaptor 1, in effect, prevents or reduces the chance of the septum of the left ventricle 19 from collapsing across the inlet of the cannula assembly 33 and occluding the cannula. The adaptor 1 also prevents or at least reduces the risk of ventricular collapse when a suction force is applied to the ventricle by the cannula.

Preferably, the adaptor 1 may be positioned within the ventricle to access the blood proximal to the centre of the ventricle. This preferred position allows the outer sides of end 1, which are inserted, to be able to be washed with fresh blood and thereby prevent thrombus formation. Additionally, the cannula when inserted into the heart should preferable not interfere with the valve of the patient's heart, in particular the mitral valve, and/or the septum wall of the heart to it's positioning.

Please note that the adaptor 1 may also be used in conjunction with either of the left or right ventricles. However, the left ventricle is preferred. Additionally, the adaptor 1 may also include a funnel shape wherein one side of the funnel is relatively longer than the opposed side (not shown in the accompanying Figs.). The longer side of this funnel may preferable be implanted against the septum wall to increase support to the septum and further reduce the risks of partial or full collapse of a portion of the heart.

The adaptor 1 may also include a plurality of holes around the base of the portion of the funnel which is inserted into the ventricle. The effect of these holes may be to reduce the risk of thrombogenesis or blood clot formation around the said portion of the funnel.

Additionally, the adaptor 1 may allow easier positioning within the ventricle of the heart, as the funnel can be positioned to remove blood from approximately centre of the ventricle.

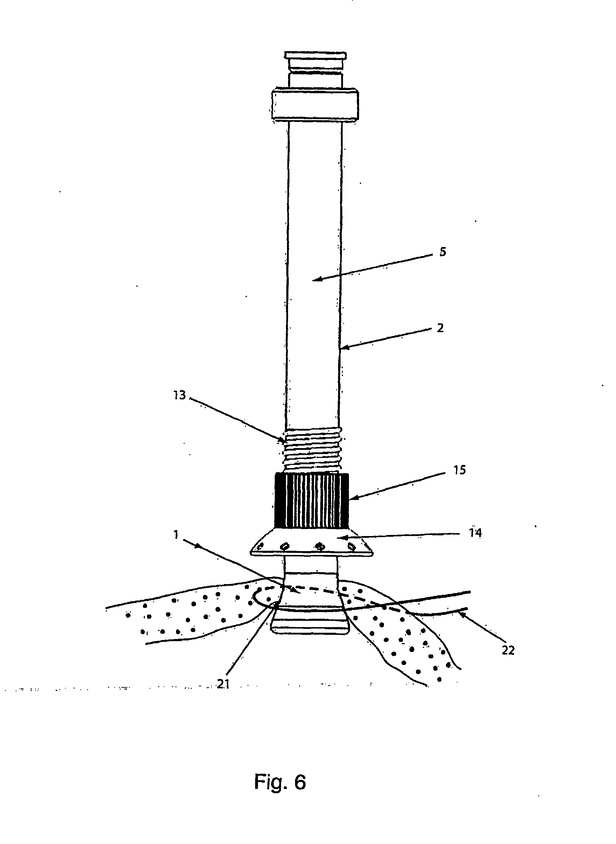

Fig. 5 also shows the insertion of the adaptor 1, of the cannula 33 of Fig. 4, into a cored hole 21 of the heart 20. Preferably, this cannula assembly would be inserted within the apex of a left ventricle 19 of the heart 20. Fig. 6 shows this preferred embodiment of the present invention being secured within said cored hole 21 by a suture 22 circumferentially securing adaptor 1.

Further to this preferred embodiment, the cannula 33 includes a thread 13 which is capable of engaging a co-operating thread (not shown) mounted on the inside sewing collar 15 and said sewing collar 15 is connected to the sewing ring 14. This means of engagement allows for the adjustability of the location of the sewing ring 14. Fig. 4 shows this embodiment wherein the sewing ring 14 is in a position relatively distal from the adaptor 1 of the cannula.

It is also further envisaged in another preferred embodiment that the thread 13 and the cooperating thread on the sewing collar 15 may be replaced with silicone adhesive. This may allow the sewing collar 15 to be more easily slid along the shank of the cannula. Once the desired position is achieved, the sewing collar 15 may also be glued in place.

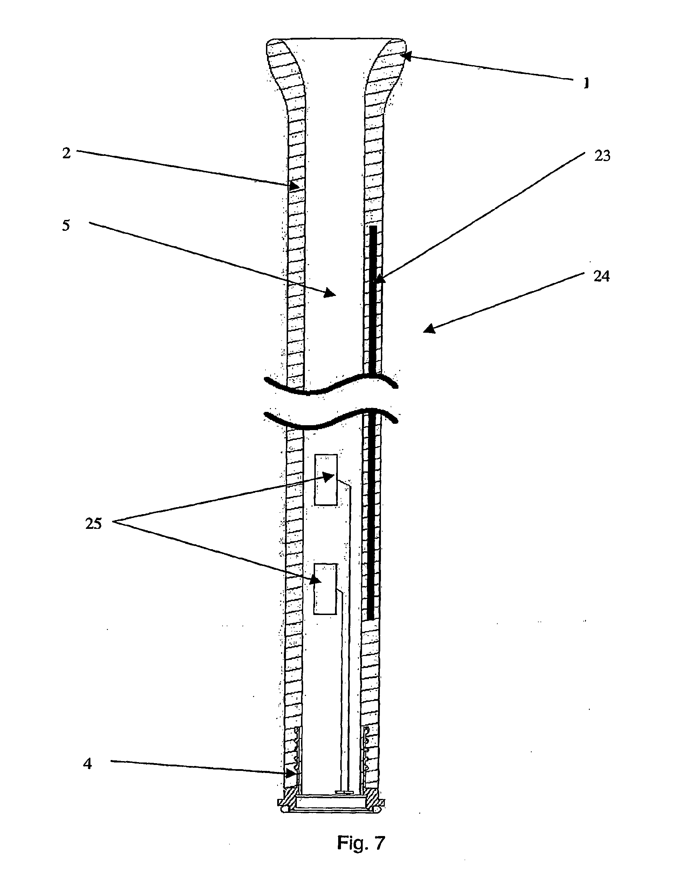

Fig. 7 shows a further embodiment 24 according to the present invention. Preferably in this embodiment, a positioning strip 23 is attached or integrally inserted within the lumen 2. This strip 23 allows for the orientation of the cannula to be determined when implantation is taking place. Alternatively, the positioning strip 23 could be replaced by a radiopaque region to allow the orientation and position of cannula to be determined by radiographic means.

Preferably, the cannula 24 illustrated in Fig. 7 includes sensors 25 within the lumen 2. These sensors 25 are capable of detecting and/or measuring blood flow rates and/or pressures within the shank of the cannula. These sensors 25 may preferably include a piezoelectric or ultrasonic detector to measure blood pressure and/or flow within the tubular shank of the embodiment. It should be understood that these sensors 25 are preferably encapsulated within the lumen 2 in such a manner that they do not contact the blood flow.

The above embodiments may also include at least one resilient pliable strip within the lumen 2 (or shank) of the cannula. This strip may enable the cannula to remain flexible whilst allowing the cannula to retain a deformed shape.

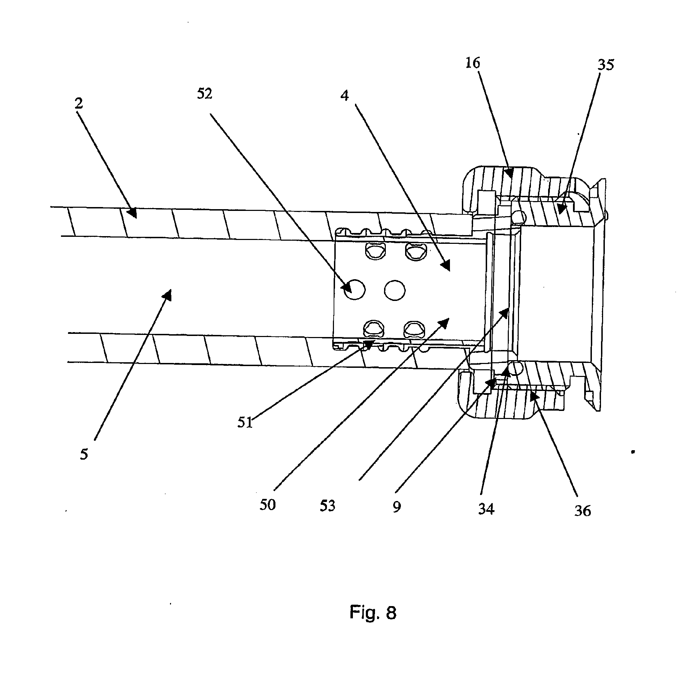

In a further preferred embodiment according to the present invention, shown by Fig. 8, a cannula assembly is connected to a blood pump. Locking nut 16 is screwed onto the mating external thread 36 of a spigot 35 on the blood pump, compressing a silicone 'O' ring 34 between a 'O' ring groove of the blood pump connector 4 and the annular outer face of the blood pump spigot 35.

The barrel portion 50 of the blood pump connector 4 preferably includes a plurality of annular ribs 51 and radially disposed holes 52. Ribs 51 and holes 52 serve to improve the mechanical bonding between the lumen 2 and the barrel portion 50. The barrel portion 50 is preferably made of a material that prevents galvanic corrosion such as titanium. Additionally, the overall wall thickness of the barrel portion 50 may be less than the wall thickness of the lumen 2 such that the cylindrical barrel portion 50 of the blood pump connector 4 preferably lies completely enclosed within the cylinder end portion of the lumen 2.

The interior surface of the lumen 2 may also form a continuous surface with the interior surface of the blood pump spigot 35. Preferably, the inner surface of lumen 2 abuts against said spigot 35 at point 53. The barrel portion 50 is preferably encased within the lumen 2 and thereby the blood channel 5 has no breaks or interruptions. The lack of breaks or interruptions may minimise thrombus formation within blood channel 5.

In yet a further preferred embodiment of the invention, the end of the cannula to be attached to the spigot of the blood pump is so formed as to include an integrally moulded locking nut shoulder and an 'O' ring configuration adapted to provide sealing means with the pump.

Alternately, the blood pump connector 4 includes a lumen that extends past the lip of the blood pump connector 4 to form an integrally moulded 'O' ring which functions to seal the interior of the cannula when connected to the blood pump (please note that this feature is not shown in the accompanying Figs.).

Also, the locking nut 16 may alternatively be replaced with some other fastener such as a snap lock connector to mate with a corresponding snap lock arrangement on the blood pump spigot.

Preferably, the shank of any of the abovedescribed embodiments may be constructed of silicone rubber. Preferably, the total wall thickness of the shank, if the shank is constructed of silicone rubber, may be between 2-4mm and the most preferred overall wall thickness being approximately 3mm.

In a further preferred embodiment as shown in Fig. 9, an alternative locking nut 37 may be joined to an end of a cannula. This embodiment of a locking nut 37 includes axial ribbing 41 covering the outer surface of nut 37 to provide grip when engaging the nut 37 against a second connector 40 mounted on the surface of the pump spigot 35. This locking nut 37 may prevent the cannula from disengaging the second connector 40, when in use.

The preferred ratchet system of Fig. 9 includes a male portion 39 positioned on the outer surface of the second connector 40 and a female portion 38 positioned on the outer surface of the locking nut 37. Preferably, the female portion may include a corrugated surface. This corrugated surface mates with the male portion 37 of said ratchet system in a manner so as to be lockable when the locking nut 37 engages with the second connector 40. This arrangement allows the nut to tighten, but not disengage without deflection of the flange 39.

Alternately, the male portion may be positioned on the outer surface of the locking nut 37 and female portion 38 may be positioned on the outer surface of the second connector 40 and this configuration may provide a similar net result to the aforementioned embodiment. The locking system may be disengaged by use of a specialised removal tool (not shown in the accompanying figures), which is adapted to engage the locking system and deflect the male portion 39 and this action, in turn, allows the release of the locking nut 37.

In another not shown embodiment a flexible reinforcing material similar to flexible reinforcing material 12 shown in Fig. 3, may be integrally moulded such that it is encapsulated within a single thick lumen similar to that shown in Fig. 1.

The above describes only some embodiments of the present invention and modifications, obvious to those skilled in the art, can be made without departing from the scope of the present invention.

## Claims

1. A cannula for the transport of blood, said cannula comprising
an elongate, flexible tubular shank defining a blood channel (5) therethrough between a first end and a second end;
the shank including a pliable, funnel-shaped adaptor (1) provided at an end of the shank which end is to be inserted in such a manner that the funnel-shaped adaptor (1) broadens toward the end of the cannula which end is to be inserted into the heart;
wherein the shank is adapted to be clamped to occlude the blood channel without substantially damaging said shank;
said shank being resistant to kinking stress.

2. The cannula of claim 1, wherein said flexible tubular shank has an outer portion having a hardness of 50 to 65 Shore.

3. The cannula of claim 2, wherein said outer portion of said tubular shank is an outer sheath (7), and said flexible tubular shank further comprises an inner sheath (32) having a substantially lesser hardness than said outer sheath.

4. The cannula of claim 3, wherein said inner sheath (32) has a hardness of about 35 Shore.

## Patentansprüche

1. Kanüle für den Transport von Blut, wobei die Kanüle folgendes umfasst:
einen länglichen, flexiblen röhrenförmigen Schaft, der eine Blutrinne (5) dadurch zwischen einem ersten und einem zweiten Ende definiert;
wobei der Schaft einen biegbaren trichterförmigen Adapter (1) umfasst, der an einem Ende des Schafts bereitgestellt ist und wobei das Ende so einzuführen ist, dass der trichterförmige Adapter (1) in der Nähe des Kanülenendes breiter wird, das in das Herz einzuführen ist;
wobei der Schaft so angeordnet ist, dass er einzuklemmen ist, um das Blut zu verschließen, ohne den Schaft im Wesentlichen zu beschädigen; wobei der Schaft beständig gegen Knickbeanspruchung ist.

2. Kanüle nach Anspruch 1, wobei der flexible röhrenförmige Schaft einen äußeren Teil hat, der eine Härte von 50 - 65 Shore hat.

3. Kanüle nach Anspruch 2, wobei der äußere Teil des röhrenförmigen Schafts ein Außenmantel (7) ist und der flexible röhrenförmige Schaft weiterhin einen Innenmantel (32) umfasst, der eine Härte im Wesentlichen kleiner als die Härte des Außenmantels hat.

4. Kanüle nach Anspruch 3, wobei der Innenmantel (32) eine Härte von etwa 35 Shore hat.

## Revendications

1. Canule pour le transport de sang, comprenant ladite canule :
un manche tubulaire, flexible, oblong définissant un conduit (5) de sang à travers le même entre un premier et un deuxième bout;
incluant le manche un adaptateur (1) pliable en forme d'entonnoir fourni à un bout du manche, devant ledit bout être inséré de façon que l'adaptateur (1) en forme d'entonnoir s'élargisse vers le bout de la canule dont le bout doit être inséré dans le coeur;
dans laquelle le manche est adapté à être fixé afin d'obstruer le sang sans essentiellement endommager ledit manche; étant ledit manche résistant à l'effort de flamblage.

2. La canule de la revendication 1, dans laquelle ledit manche tubulaire flexible a une partie extérieure ayant une dureté de 50 à 65 Shore.

3. La canule de la revendication 2, dans laquelle ladite partie extérieure dudit manche tubulaire est une gaine extérieure (7), et ledit manche tubulaire flexible comprend en outre une gaine intérieure (32) ayant une dureté essentiellement inférieure que ladite gaine extérieure.

4. La canule de la revendication 3, dans laquelle ladite gaine intérieure (32) a une dureté d'environ 35 Shore.
